# EUROPEAN PATENT APPLICATION

(11) **EP 4 491 168 A1**
(43) Date of publication of application: **15.01.2025**
(21) Application number: 23766858.7
(22) Date of filing: 07.03.2023
(51) Int. Cl.: A61K 6/836

(54) **DENTAL GLASS COMPOSITION**

(30) Priority: 07.03.2022 JP 2022034827
(71) Applicant: Kuraray Noritake Dental Inc., Kurashiki-shi, Okayama 710-0801 (JP)
(72) Inventor: EMOTO, Tomohiro, Miyoshi-shi, Aichi 470-0293 (JP); KASHIKI, Nobusuke, Miyoshi-shi, Aichi 470-0293 (JP)
(74) Representative: D Young & Co LLP
(86) International application number: PCT/JP2023/008644
(87) International publication number: WO 2023/171682

(57) **Abstract**

The present invention provides a dental glass composition that exhibits excellent fixability to dental prostheses, and does not require fixation firing, in addition to reducing notable bubble formation and interface delamination at the interface between the base material and dental porcelain without fixation firing, enabling the build-up and firing of dental porcelain on the dental glass composition. The present invention relates to a dental glass composition comprising a glass powder (A) and a binder, wherein the binder comprises a hydrophilic organic compound (B) that is solid at 25°C, and the dental glass composition is essentially free of water and propylene glycol.

## Description

### TECHNICAL FIELD

The present invention relates to dental glass compositions used for staining and shade adjustment of dental prostheses. More specifically, the invention relates to dental glass compositions suitable for use in, for example, crowns, inlays, bridges, implant superstructures, denture artificial teeth employing ceramic materials. Particularly, the invention relates to dental glass compositions (hereinafter, also referred to as "porcelain stains") suited as compositions for adjusting the shade of ceramic crown porcelain.

### BACKGROUND ART

Ceramic dental prostheses, such as metal-ceramic dental prostheses and all-ceramic dental prostheses, are a type of dental prostheses (for example, veneer crowns, crowns, and post crowns) used to restore aesthetics similar to natural teeth. These dental prostheses utilize dental porcelain to express the dentin and enamel shades on various materials such as metal, zirconium oxide (zirconia), aluminum oxide (alumina), feldspar glass, and disilicate glass.

In the production method of ceramic dental prostheses, a technique is available that is aimed at mimicking the shade of individual natural teeth. This involves expressing features of natural teeth in dental prostheses, such as the white band, the coloration of dentin, cervical region, and adjacent surfaces, as well as hairlines and enamel cracks. To achieve this, for example, porcelain stains are applied to the dental prosthesis, allowing the ceramic dental prosthesis to blend more naturally with the shade of the natural teeth surrounding the teeth where the dental prosthesis is applied.

When using porcelain stains, the process involves taking a porcelain stain of the appropriately selected shade onto a mixing glass plate or stain pallet. Porcelain stains are typically available in either powder or paste form. When the porcelain stain is a powder, an appropriate amount is taken onto a mixing glass plate or stain pallet. This must be followed by adding water or a specialized solution to make the powder applicable, and kneading it into a paste form by achieving the desired paste properties through adjustments such as by adjusting the powder-to-liquid ratio. When the porcelain stains are paste form, the paste properties undergo changes such as an increase in viscosity over time during storage or after taking an appropriate amount of paste onto a mixing glass plate or stain pallet. This makes it difficult to apply the paste during use, posing applicability issues such as smudging during application. There accordingly is a need for porcelain stains that are easy to work with and demonstrate superior applicability.

In order to mimic natural teeth, two techniques are commonly employed in staining ceramic dental prostheses: the internal staining method, where porcelain stains are sandwiched between enamel-expressing porcelain and dentin-expressing porcelain, and the surface staining method, where porcelain stains are applied directly to the outermost surface of enamel-expressing porcelain.

These methods require firing porcelain stains with a dental laboratory porcelain furnace at the optimum firing schedule for each porcelain stain after it is applied to the dental prosthesis (hereinafter, also referred to as "fixation firing"), in order to allow the inorganic pigment components contained in the porcelain stain to fuse into the dental prosthesis, and, in the case of the internal staining method, to avoid affecting subsequent porcelain build-up with another porcelain. Because the production of dental prostheses requires multiple firings, there is a need for porcelain stains that can shorten the production process.

As a shade adjustment composition for ceramic crown porcelain applications, for example, Patent Literature 1 discloses a powder- or paste-form porcelain stain comprising specific aluminosilicate glass. Patent Literature 2 discloses a paste that eliminates the need for pre-firing before applying a body porcelain layer. This is achieved by brush coating a dental coping with a coating material comprising an opaque porcelain powder and an aqueous colloidal dispersion of urethane polymer before applying the body porcelain layer, and air-drying the coating for 20 minutes.

### CITATION LIST

### Patent Literature

Patent Literature 1: JP 2009-207743 A
Patent Literature 2: JP S59-196807 A

### SUMMARY OF INVENTION

### Technical Problem

However, Patent Literature 1 requires applying the porcelain stain in paste form. This means that a paste must be prepared by mixing powder with liquid (for example, water) every time before use. Additionally, it is difficult to adjust the paste because the paste cannot be easily applied when it is too viscous, whereas bleeding tends to occur when the viscosity is too low. Moreover, when using the paste-form porcelain stain described in Patent Literature 1, there is an issue where its viscosity increases over time, altering the paste properties and making the paste difficult to apply.

Patent Literature 1 also requires firing for each application when forming multiple layers, regardless of whether the internal staining method or surface staining method is employed. Another issue is that the internal staining method requires firing and fixing the porcelain stain before building up porcelain on the surface layer, resulting in an increased number of steps. If the fixation firing is skipped, it may lead to the porcelain stain unintentionally mixing with the porcelain in the subsequent porcelain build-up process, or the absence of fixing may cause the color of the porcelain stain to flow out before or during the firing of the layered porcelain.

In Patent Literature 2, the urethane polymer contained cannot be fully burned off under the porcelain firing conditions, leading to carbonization or bubble formation. Achieving even application is also difficult with the technique described in Patent Literature 2. The generation of bubbles, in particular, presents a problem as it adversely affects the fusing between the porcelain and the coping (frame / base / substrate). Additionally, Patent Literature 2 requires a 20-minute air drying period, longer than the typical duration (5 to 8 minutes) required for this process, making it impractical in terms of processing time.

It is accordingly an object of the present invention to provide a dental glass composition that exhibits excellent fixability to dental prostheses, allows for layering of color without the need for fixation firing, and can reduce notable bubble formation and interface delamination at the interface between the base material and dental porcelain without fixation firing, enabling the build-up and firing of dental porcelain on the dental glass composition.

### Solution to Problem

The present inventors conducted intensive studies to find a solution to the foregoing issues, and found that a dental glass composition that comprises a glass component and a hydrophilic organic compound that is solid at 25°C, and in which water and propylene glycol are essentially absent can be directly applied to dental prostheses without the need to prepare a paste, and enables the creation of a dental prosthesis in the desired shade with reduced bubble formation, even without fixation firing required by the internal staining method. This led to the completion of the present invention after further examinations.

The present invention includes the following.
[1] A dental glass composition comprising a glass powder (A) and a binder,
   wherein the binder comprises a hydrophilic organic compound (B) that is solid at 25°C, and
   the dental glass composition is essentially free of water and propylene glycol.
[2] The dental glass composition according to [1], wherein the glass powder (A) has an average particle diameter of 0.05 µm to 50 µm.
[3] The dental glass composition according to [1] or [2], wherein the hydrophilic organic compound (B) has a melting point of 35°C or more.
[4] The dental glass composition according to any one of [1] to [3], wherein the hydrophilic organic compound (B) is a water-soluble polymer.
[5] The dental glass composition according to any one of [1] to [4], wherein the content of the hydrophilic organic compound (B) is 1 to 50 mass%.
[6] The dental glass composition according to any one of [1] to [5], wherein the binder further comprises an unsaturated fatty acid component (C) that is liquid at 25°C.
[7] The dental glass composition according to [6], wherein the content of the unsaturated fatty acid component (C) is 0.5 to 30 mass%.
[8] The dental glass composition according to any one of [1] to [7], wherein the binder further comprises a wax component (D) that is solid at 25°C.
[9] The dental glass composition according to any one of [1] to [8], wherein the content of the water and propylene glycol is less than 1,000 ppm by mass.
[10] The dental glass composition according to any one of [1] to [9], which is in solid form at 25°C.
[11] The dental glass composition according to any one of [1] to [10], which is a porcelain stain.
[12] A method for producing a dental prosthesis, comprising the steps of:
   applying a dental glass composition of any one of [1] to [11] to a dental prosthesis base material having a ceramic surface; and
   conducting firing after building up dental porcelain, without firing the base material following the application of the dental glass composition and before building up dental porcelain.
[13] The method for producing a dental prosthesis according to [12], wherein the firing step after building up dental porcelain involves a firing temperature of 700 to 1,100°C.

### Advantageous Effects of Invention

According to the present invention, a dental glass composition can be provided that exhibits excellent fixability to dental prostheses, allows for layering of color without the need for fixation firing, and can reduce notable bubble formation and interface delamination at the interface between the base material and dental porcelain without fixation firing, enabling the build-up and firing of dental porcelain on the dental glass composition.

A dental glass composition of the present invention exhibits excellent fixability without requiring fixation firing even when applied to form multiple layers, preventing mixing with dental porcelain in the subsequent porcelain build-up process, thereby reducing the flowing of its color before or during the firing of the layered dental porcelain.

Because there is no need for fixation firing, a dental glass composition of the present invention can shorten the production process, providing industrial advantages.

A dental glass composition of the present invention also exhibits superior chromogenicity, allowing for layering of color and adjustment to the desired color tone.

Additionally, a dental glass composition of the present invention undergoes reduced changes over time in the glass composition itself, enhancing its workability and allowing for thin application on dental prostheses while also being applicable without bleeding due to its non-flowing nature.

Moreover, a dental glass composition of the present invention can be applied alone to dental prostheses, or provide a gradation from the cervical to incisal region using a brush or a sponge such as an eye color tip applicator, or produce blurring effects with fingers, enabling the creation of dental prostheses having desired shades, both easily and with good workability.

It is also possible to shorten the dental prosthesis production process because a dental glass composition of the present invention enables the porcelain building up process to be carried out without prior fixation firing while reducing bubble formation.

### BRIEF DESCRIPTION OF DRAWINGS

[FIG. 1] A photograph showing a specimen subjected to fixability and chromogenicity evaluation according to Example 1.
[FIG. 2] A photograph showing a specimen subjected to fixability and chromogenicity evaluation according to Comparative Example 5.
[FIG. 3] A diagram showing a control cut surface after building up and firing dental porcelain on a base material using the conventional internal staining method (with fixation firing), along with a light micrograph of the cut surface.
[FIG. 4] A light micrograph of a cut surface in the evaluation of interface condition according to Example 1.
[FIG. 5] A light micrograph of a cut surface in the evaluation of interface condition according to Comparative Example 2

### DESCRIPTION OF EMBODIMENTS

A dental glass composition of the present invention comprises a glass powder (A) and a binder, wherein the binder comprises a hydrophilic organic compound (B) that is solid at 25°C, and the dental glass composition is essentially free of water and propylene glycol.

### <Glass Powder (A)>

The glass powder (A) contained in a glass composition of the present invention is described first. The glass powder (A) used in the present invention is not particularly limited as long as the present invention can exhibit its effects, and may be any of common dental glass powders that can be fused into base materials (materials used in dental prostheses, such as cores or frames).

Examples of the glass powder (A) include potassium aluminosilicate glass (amorphous-type potassium aluminosilicate glass (4SiO₂·Al₂O₃·K₂O), leucite crystal-type potassium aluminosilicate glass), fluoroapatite glass, and lithium silicate glass. Examples of the constituent components of the glass powder (A) include SiO₂, Al₂O₃, B₂O₃, Li₂O, Na₂O, K₂O, CaO, MgO, and Sb₂O₃. Depending on the base material being used, multiple components are selected in appropriate proportions.

The glass powder (A) may also incorporate components such as TiO₂, SrO, BaO, ZnO, CeO₂, ZrO₂, and P₂O₅ in appropriate proportions. The glass powder (A) may be used alone, or two or more thereof may be used in combination.

Preferably, the glass powder (A) used in the present invention has a coefficient of linear thermal expansion similar to that of the base material used. For example, the glass powder (A) may have a coefficient of linear thermal expansion of 6.0 × 10⁻⁶K⁻¹ to 14.0 × 10⁻⁶K⁻¹ in a 25°C to 500°C range. The coefficient of linear thermal expansion can be measured using known methods and devices. For example, the coefficient of linear thermal expansion can be measured by heating a sample from room temperature to 500°C using a thermal analyzer (TMA120 manufactured by Seiko Instruments Inc. under this trade name; a rate of temperature increase: 5°C/min).

The glass powder (A) used in the present invention can be made from a broad range of commonly used and known ceramic materials. A broad range of ceramic raw materials can be used for the glass powder (A) with no restrictions on the glass composition, as long as the glass powder (A) is in powder form. This is because a dental glass composition of the present invention, by combining and integrating the powdery glass with the binder comprising a hydrophilic organic compound (B), exhibits superior fixability and excellent adhesion at the interface between the base material and dental porcelain as a whole, and does not undergo changes in fixability and interface adhesion due to the glass composition (presence or absence of specific oxides). This allows the glass powder (A) to employ ceramic raw materials that exhibit chromogenicity in the desired shade. The raw materials are not particularly limited, as long as these are specific components themselves such as above, and/or substances that can transform into these components upon heating in the atmosphere. After determining the desired glass composition through calculation, these materials are mixed by adjusting their proportions accordingly.

There are no particular restrictions on the method of mixing the raw material substances of the glass, and known methods and devices can be used. It is preferable to evenly disperse the raw material substances of glass powder (A) through mixing.

After being mixed in this fashion, the raw material substances are melted at 1 ,200°C or more, and the resultant melt is cooled and pulverized to yield a glass powder. The method used to melt the raw material substances is not particularly limited, and may employ known methods and devices. There are no particular restrictions on the method of melting the raw material substances, as long as the mixed raw material substances completely melt and uniformly form an amorphous material, without causing unwanted effects such as sublimation of the components.

The method of cooling the melt is not particularly limited either, and may employ known methods and devices. The cooling process for the melt may involve processes such as rapid cooling in water. The resulting clump of glass is dried and pulverized to obtain glass powder (A). There are no particular restrictions on the method of pulverizing the glass clump, and known methods and devices can be used. The classification process to achieve the desired grain size is not limited to particular methods, and this may be achieved by using known methods and devices.

The glass powder (A) used in the present invention may comprise a colorant. The colorant may be amorphous or crystalline. The colorant is not limited to particular forms, and may have a powder form. In certain embodiments, the colorant may comprise a crystalline powder. Examples of the colorant include pigments and fluorescent agents. The colorant may be used alone, or two or more thereof may be used in combination. A broad range of colorants can be used for the glass powder (A) with no particular restrictions on the crystal system or its variety. This is because a dental glass composition of the present invention, by combining and integrating the powdery glass with the binder comprising a hydrophilic organic compound (B), exhibits superior fixability and excellent adhesion at the interface between the base material and dental porcelain as a whole, and does not undergo changes in fixability and interface adhesion due to the type of colorant (presence or absence of specific oxides).

The pigments are preferably inorganic pigments. Examples of the inorganic pigments include oxides of at least one element selected from the group consisting of Ti, V, Cr, Mn, Fe, Co, Ni, Zn, Y, Zr, Sn, Sb, Bi, Ce, Pr, Sm, Eu, Gd, Tb, and Er. Preferably, the inorganic pigments comprise oxides of at least one element selected from the group consisting of Ti, V, Cr, Mn, Fe, Co, Ni, Pr, Tb, and Er. Examples of such oxides include praseodymium oxide, vanadium oxide, iron oxide, nickel oxide, chromium oxide, manganese oxide, cerium oxide, tin oxide, zirconium oxide, zinc oxide, and titanium oxide.

The inorganic pigments may be composite pigments. Examples of the composite pigments include (Zr,V)O₂, Fe(Fe,Cr)₂O₄, (Ni,Co,Fe)(Fe,Cr)₂O₄·ZrSiO₄, and (Co,Zn)Al₂O₄. Preferably, the composite pigments comprise (Zr,V)O₂.

Examples of the fluorescent agents include Y₂SiO₅:Ce, Y₂SiO₅:Tb, (Y,Gd,Eu)BO₃, Y₂O₃:Eu, YAG:Ce, ZnGa₂O₄:Zn, and BaMgAl₁₀O₁₇:Eu.

When the glass powder (A) comprises a colorant, the glass powder (A) may be one resulting solely from mixing of the colorant with a raw material glass powder containing no colorant, or may be one pulverized into fine particles after the colorant and a raw material glass powder containing no colorant are mixed and fused under heat at 600 to 1,200°C.

Considering the need to ensure a balance between the smallest possible grain size and a certain level of coarseness to produce color in achieving shades similar to natural teeth, it is preferable for the glass powder (A) used in the present invention to have an average particle diameter of 0.05 to 50 µm or less, more preferably 0.08 to 35 µm, even more preferably 0.1 to 20 µm, particularly preferably 0.1 to 10 µm, most preferably 0.1 to 5 µm. When the average particle diameter is 50 µm or less, the dental glass composition can sufficiently adhere to the frame, preventing it from falling off while building up porcelain, and ensuring superior aesthetic quality with no visible color particles. When average particle diameter is 0.05 µm or more, the glass powder (A) will not be burned off due to firing, enabling sufficient color production.

The average particle diameter of glass powder (A) can be measured using a laser diffraction scattering method. As an example of the laser diffraction scattering method, the average particle diameter of glass powder (A) can be measured by volume using a laser diffraction particle size distribution analyzer (SALD-2300, manufactured by Shimadzu Corporation). The average particle diameter of glass powder (A) refers to the median diameter (d₅₀), where cumulative volume frequency in the volumetric particle size distribution reaches 50% from the smaller diameter side.

In a dental glass composition of the present invention, the preferred content of glass powder (A) is such that it allows for kneading with the hydrophilic organic compound (B). The content of glass powder (A) is preferably 25 mass% or more in the total amount of the dental glass composition. In view of chromogenicity and improved fixability to dental prostheses, the content of glass powder (A) is more preferably 35 mass% or more, even more preferably 45 mass% or more, particularly preferably 55 mass% or more. The content of glass powder (A) is preferably 97 mass% or less, more preferably 95 mass% or less, even more preferably 93 mass% or less, particularly preferably 91 mass% or less, though it is not particularly limited.

In an embodiment where the binder comprises a wax component (D) in addition to the hydrophilic organic compound (B), the content of glass powder (A) is preferably 25 mass% or more in the total amount of the dental glass composition. In view of chromogenicity and improved fixability to dental prostheses, the content of glass powder (A) is more preferably 35 mass% or more, even more preferably 45 mass% or more, particularly preferably 55 mass% or more. In such embodiments, the content of glass powder (A) is preferably 95 mass% or less, more preferably 93 mass% or less, even more preferably 89 mass% or less, particularly preferably 85 mass% or less, though it is not particularly limited.

In a dental glass composition of the present invention, the glass powder (A) may employ commercially available dental porcelain.

Such commercially available dental porcelain is not particularly limited, as long as it is dental staining porcelain. Examples of commercially available dental staining porcelain products include various types of surface stains and internal stains, such as Cerabien^{®} ZR internal stain / external stain, and Cerabien^{®} ZR press LF internal stain / external stain (manufactured by Kuraray Noritake Dental Inc.).

### <Hydrophilic Organic Compound (B) that is Solid at 25°C>

The following describes the hydrophilic organic compound (B) that is solid at 25°C (hereinafter, also referred to simply as "hydrophilic organic compound (B)") contained in a dental glass composition of the present invention.

The binder in a dental glass composition of the present invention comprises a hydrophilic organic compound (B).

The binder means a substance that possesses the ability to bond a base material and dental porcelain.

The hydrophilic organic compound (B) is incorporated to disperse the glass powder (A) and solidify the dental glass composition at 25°C. The presence of hydrophilic organic compound (B) enhances the adhesion and fixability to the base material, allowing for layering of color without necessitating fixation firing. It is also possible to reduce bubble formation at the interface between the base material and dental porcelain when building up and firing dental porcelain on the dental glass composition. The hydrophilic organic compound (B) may be used alone, or two or more thereof may be used in combination.

In view of dispersing the glass powder (A) and solidifying the dental glass composition at 25°C, the hydrophilic organic compound (B) used in the present invention preferably comprises those that are solid at 25°C and melt upon heating.

In this specification, the phrase "solid at 25°C" encompasses not only substances that are solid at 25°C but those that are semi-solid such as pastes. Dental glass compositions that have solidified at 25°C may be semi-solid such as pastes.

The heating temperature for melting the hydrophilic organic compound (B) can vary depending on the type of hydrophilic organic compound (B), and may be 50°C or more, 80°C or more, 100°C or more, or 150°C or more, though it is not particularly limited.

A certain preferred embodiment is, for example, a dental glass composition that comprises a glass powder (A) and a binder, and in which the binder comprises a hydrophilic organic compound (B), and the hydrophilic organic compound (B) consists of a material that is solid at 25°C and melts upon heating.

Using materials that are solid at 25°C and melt upon heating has advantages over related art in terms of preventing changes in properties such as a viscosity increase in the glass composition over time, and eliminating the need for repeated fixation firings by not requiring fixation firing. This allows a dental glass composition of the present invention to enable easy layering of color, and building up of porcelain (for example, such as surface staining dental porcelain) thereon. Additionally, it is possible to allow a dental glass composition of the present invention to be applied in a thin layer.

Hydrophilic organic compound (B) refers to an organic compound with a solubility of 1 mass% or more in water at 25°C, preferably one with a solubility of 5 mass% or more, more preferably 10 mass% or more, even more preferably 20 mass% or more, particularly preferably 30 mass% or more in water at 25°C. With a solubility of 1 mass% or more in water at 25°C, the adhesion and fixability to the base material increase, allowing for layering of color without needing fixation firing, and reducing bubble formation when building up and firing dental porcelain on the dental glass composition. Taking these factors into account, the hydrophilic organic compound (B) is not particularly limited, and may employ a wide range of compounds, provided that it is solid at 25°C, and can melt when heated to a specific melting point while being hydrophilic in nature, because such compounds can improve the dispersibility of glass powder (A) and the adhesion and fixability to the base material, and produce an effect that reduces bubble formation.

In view of storage stability to stably maintain a solid state at room temperature and from the perspective of achieving good fixability to dental prostheses, the melting point of hydrophilic organic compound (B) is preferably 35°C or more, more preferably 40°C or more, even more preferably 45°C or more, particularly preferably 50°C or more. When the melting point is less than 35°C, it may not be possible to maintain a solid form, raising a possibility of flowing out from the drawn surface while layering the dental glass composition or building up the surface porcelain layer. The melting point of the hydrophilic organic compound (B) is preferably 120°C or less, more preferably 105°C or less, even more preferably 95°C or less, particularly preferably 85°C or less. A melting point higher than 120°C poses a risk of failed adhesion to dental prostheses and inadequate coloring.

The hydrophilic organic compound (B) may employ known compounds, preferably water-soluble polymers.

Examples of water-soluble polymers include cationic water-soluble polymers, anionic water-soluble polymers, amphoteric water-soluble polymers, and non-ionic water-soluble polymers.

Examples of the cationic water-soluble polymers include water-soluble polymers comprising at least one selected from the group consisting of polyethyleneimine, polyallylamine, polyvinylamine, dimethylaminoethyldextran, and an imidazole group.

Examples of the anionic water-soluble polymers include polysaccharides such as xanthan gum, guar gum, carrageenan, tamarind seed gum, gum arabic, locust bean gum, pectin, sodium starch glycolate, sodium starch phosphate, alginate salts (such as sodium alginate), propylene glycol alginate, propylene glycol alginate ester, hyaluronan, chondroitin sulfate, dermatan sulfate, keratan sulfate, heparan sulfate, and salts of these. Other examples include polyacrylic acid, and salts of polyacrylic acid (such as sodium polyacrylate, and ammonium polyacrylate), carboxyvinyl polymer, and polymaleic acid.

Examples of the amphoteric water-soluble polymers include proteins or amino acids such as gelatin, collagen, casein, and gluten, and their derivatives.

Examples of the non-ionic water-soluble polymers include cellulose lower alkyl ether compounds such as methyl cellulose, ethyl cellulose, carboxycellulose, carboxymethyl cellulose, sodium carboxymethyl cellulose, calcium carboxymethyl cellulose, hydroxymethyl cellulose, hydroxyethyl cellulose, and hydroxypropyl cellulose; vinyl compounds such as polyvinylpyrrolidone, and polyvinyl alcohol; polysaccharides such as pullulan and starch, and their derivatives; and polyacrylamides such as polyglycosyloxyethyl methacrylate. Other examples include polyalkylene glycol compounds such as polyethylene oxide, polyethylene glycol, polypropylene glycol, and block copolymers of polyethylene glycol and polypropylene glycol.

In this specification, polyethylene glycol refers to polymers with a weight-average molecular weight (Mw) of 20,000 or less. Polyethylene oxide refers to polymers with a weight-average molecular weight (Mw) of more than 20,000, preferably 50,000 or more.

In order to produce enhanced chromogenicity due to improved stability of glass powder (A), the hydrophilic organic compound (B) is more preferably a non-ionic water-soluble polymer, even more preferably a polyalkylene glycol compound, particularly preferably polyethylene glycol (PEG). In this specification, PEG is also referred to as macrogol.

The weight-average molecular weight (Mw) of water-soluble polymers representing the hydrophilic organic compound (B) is not particularly limited, and may be, for example, 20,000 or less. The weight-average molecular weight (Mw) of water-soluble polymers representing the hydrophilic organic compound (B) is preferably 700 or more, more preferably 750 or more, even more preferably 850 or more, particularly preferably 900 or more. The weight-average molecular weight (Mw) of water-soluble polymers representing the hydrophilic organic compound (B) may be, for example, 700 or more and 20,000 or less.

In another embodiment, the weight-average molecular weight (Mw) of water-soluble polymers representing the hydrophilic organic compound (B) may be more than 20,000. The weight-average molecular weight (Mw) of water-soluble polymers representing the hydrophilic organic compound (B) may be 2,500,000 or less, 1,500,000 or less, 1,000,000 or less, 300,000 or less, or 200,000 or less.

In this specification, the weight-average molecular weight (Mw) refers to the weight-average molecular weight determined through gel permeation chromatography (GPC) in terms of polystyrene. This measurement can be conducted using known methods.

In yet another embodiment, the number-average molecular weight (Mn) of water-soluble polymers representing the hydrophilic organic compound (B) is preferably 400 or more, more preferably 500 or more, even more preferably 550 or more. The number-average molecular weight (Mn) of water-soluble polymers representing the hydrophilic organic compound (B) may be 40,000 or less, 20,000 or less, or 10,000 or less.

In this specification, the number-average molecular weight (Mn) refers to the number-average molecular weight calculated from the hydroxyl number measured in compliance with JIS K 1557-1: 2007. This measurement can be conducted using known methods.

In view of stably maintaining a solid state at room temperature and achieving good fixability to dental prostheses, the content of the hydrophilic organic compound (B) used in the present invention is preferably 1 to 50 mass%, more preferably 3 to 45 mass%, even more preferably 5 to 40 mass%, particularly preferably 7 to 35 mass% in the total amount of the dental glass composition. When the binder comprises the hydrophilic organic compound (B) and the wax component (D), the content of hydrophilic organic compound (B) is preferably 1 to 40 mass%, more preferably 2.5 to 35 mass%, even more preferably 4 to 30 mass%, particularly preferably 6 to 25 mass% in the total amount of the dental glass composition.

A dental glass composition of the present invention is essentially free of water and propylene glycol. In this specification, the phrase "essentially free of a certain component" means that the content is less than 10 mass%, preferably less than 1 mass%, more preferably less than 0.1 mass% (less than 1,000 ppm by mass), even more preferably less than 100 ppm by mass, yet more preferably 10 ppm or less by mass, particularly preferably 1 ppm or less by mass, still more preferably 0.1 ppm or less by mass, most preferably 0 ppm by mass, unless otherwise specified with a numerical value. An example of such embodiments is a dental glass composition in which the contents of water and propylene glycol are less than 10 mass% each.

By being essentially free of water and propylene glycol, the dental glass composition can exhibit excellent stability in its properties during prolonged storage while allowing for easy layering of color with no bleeding, enabling the build-up of porcelain thereon. In certain instances, it is also possible to reduce bubble formation at the interface between the base material and dental porcelain during firing.

A dental glass composition of the present invention may optionally comprise additional components that are liquid at 25°C (hereinafter, also referred to as "additional liquid components"), aside from the unsaturated fatty acid component (C) described below.

Such additional liquid components may be used alone, or two or more thereof may be used in combination. Examples of the additional liquid components include organic solvents. Examples of the organic solvents include:
non-aromatic hydrocarbon solvents, for example, such as alkanes (e.g., pentane, hexane, heptane, octane, nonane, decane, dodecane, isododecane, and tridecane), cyclohexane, methylcyclohexane, decahydronaphthalene, and liquid paraffin;
aromatic hydrocarbon solvents, for example, such as benzene, toluene, xylene, diethylbenzene, mesitylene, tetralin, indene, naphthalene, and methylnaphthalene;
halogenated hydrocarbon solvents, for example, such as dichloromethane, dichloroethane, chloroform, and chlorobenzene;
alcohol solvents, for example, such as monohydric alcohols (e.g., ethanol, propanol, 1-butanol, 2-butanol, 1-heptanol, 2-heptanol, 3-heptanol, 1-hexanol, 2-hexanol, benzyl alcohol, 3-(benzyloxy)-1-propanol, 2-(benzyloxy)-1-butanol, 5-(benzyloxy)-1-pentanol, and oleyl alcohol), and polyhydric alcohols (e.g., ethylene glycol, polyethylene glycol, polypropylene glycol, and glycerin);
ether solvents, for example, such as diethyl ether, diisopropyl ether, methyl t-butyl ether, diisoamyl ether, ethylene glycol derivatives (e.g., monoglyme (ethylene glycol dimethyl ether), methyl cellosolve, diethyl cellosolve, diglyme, diethylene glycol monoethyl ether, diethylene glycol diethyl ether, triglyme, tetraglyme, diethylene glycol monobutyl ether, triethylene glycol monomethyl ether, ethylene glycol monobenzyl ether, ethylene glycol monophenyl ether, diethylene glycol monobenzyl ether, and poly(ethylene glycol)monomethyl ether), propylene glycol derivatives (e.g., 3-hexanolpropylene glycol monopropyl ether, dipropylene glycol monoethyl ether, tripropylene glycol monomethyl ether), 1,1-dimethoxycyclohexane, phenetole, veratrole, dioxane, and tetrahydrofuran;
ester solvents, for example, such as ethyl acetate, butyl acetate, isopropyl acetate, 3-methoxy-3-methylbutyl acetate, dimethyl carbonate, diethyl malonate, ethylene carbonate, propylene carbonate, γ-butyrolactone, and α-acetyl-γ-butyrolactone;
ketone solvents, for example, such as acetone, methyl ethyl ketone, methyl isobutyl ketone, diisobutyl ketone, acetophenone, propiophenone, and isophorone;
sulfur-containing solvents, for example, such as dimethyl sulfoxide, sulfolane, and diphenyl sulfide; and
nitrogen-containing solvents, for example, such as amide solvents (e.g., N,N-dimethylformamide, N,N-dimethylacetamide, N-methylpyrrolidone, 1,3-dimethyl-2-imidazolidinone, N,N-dimethylacrylamide, N,N-dimethylacetoacetamide, N,N-diethylformamide, N,N-diethylacetamide, hexamethylphosphoramide, and methylpyrrolidone), amine solvents (e.g., butylamine, hexylamine, octylamine, 3-methoxypropylamine, 2-methylbutylamine, and triethanolamine), nitrile solvents (e.g., acetonitrile, and benzonitrile), nitro solvents (e.g., nitrobenzene, and o-nitrotoluene), quinoline, tetrahydroquinoline, and dimethylimidazolidinone.

A certain embodiment is, for example, a dental glass composition that is essentially free of water, ethylene glycol, propylene glycol, polyethylene glycol, polypropylene glycol, and glycerin.

Another embodiment is, for example, a dental glass composition that is essentially free of water and a polyhydric alcohol. By being essentially free of the specific components, the dental glass composition in such embodiments can exhibit excellent stability in its properties during prolonged storage while allowing for easy layering of color with no bleeding, enabling the build-up of porcelain thereon. In certain instances, it is also possible to reduce bubble formation at the interface between the base material and dental porcelain during firing.

Yet another embodiment is, for example, a dental glass composition that is essentially free of water and an alcohol compound. By being essentially free of water and an alcohol compound, the dental glass composition in such embodiments can exhibit excellent stability in its properties during prolonged storage while allowing for easy layering of color with no bleeding, enabling the build-up of porcelain thereon. It is also possible to reduce bubble formation at the interface between the base material and dental porcelain during firing.

The content of the alcohol compound is preferably less than 10 mass%, more preferably 8 mass% or less, even more preferably 5 mass% or less, particularly preferably 3 mass% or less, most preferably 1 mass% or less. In such embodiments, the content of the alcohol compound may be less than 0.1 mass%, less than 100 ppm by mass, 10 ppm or less by mass, 1 ppm or less by mass, 0.1 ppm or less by mass, or 0 ppm by mass.

Still another embodiment is, for example, a dental glass composition that is also essentially free of an amine compound. By being essentially free of an amine compound, the dental glass composition can exhibit excellent stability in its properties during prolonged storage while allowing for easy layering of color with no bleeding, enabling the build-up of porcelain thereon. It is also possible to reduce bubble formation at the interface between the base material and dental porcelain during firing.

The content of the amine compound is preferably less than 10 mass%, more preferably 8 mass% or less, even more preferably 5 mass% or less, particularly preferably 3 mass% or less, most preferably 1 mass% or less. In such embodiments, the content of the amine compound may be less than 0.1 mass%, less than 100 ppm by mass, 10 ppm or less by mass, 1 ppm or less by mass, 0.1 ppm or less by mass, or 0 ppm by mass.

In embodiments free of water, alcohol compounds, and amine compounds, the dental glass composition, by the absence of water and a polyhydric alcohol, can exhibit excellent stability in its properties during prolonged storage while allowing for easy layering of color with no bleeding, enabling the build-up of porcelain thereon. In certain instances, it is also possible to reduce bubble formation at the interface between the base material and dental porcelain during firing.

Yet another embodiment is, for example, a dental glass composition that is essentially free of components that are liquid at 25°C, aside from unsaturated fatty acid component (C). The phrase "essentially free of a certain component" is as described above. An example of such an embodiment is a dental glass composition comprising less than 1 mass% of components that are liquid at 25°C, aside from unsaturated fatty acid component (C).

### <Unsaturated Fatty Acid Component (C)>

It is preferable in a dental glass composition of the present invention that the binder further comprise an unsaturated fatty acid component (C). Examples of the unsaturated fatty acid component (C) include an unsaturated fatty acid (C-1) and an unsaturated fatty acid ester (C-2). The unsaturated fatty acid component (C) may be used alone, or two or more thereof may be used in combination.

The unsaturated fatty acid component (C) is liquid at 25°C. By additionally comprising an unsaturated fatty acid component (C), a dental glass composition of the present invention can enhance its smoothness, improving workability. With the unsaturated fatty acid component (C), the dental glass composition can also have reduced hygroscopicity, exhibiting excellent stability in its properties during prolonged storage. The choice of unsaturated fatty acid component (C) is not particularly limited, and the unsaturated fatty acid component (C) can produce desired effects such as adjusting the viscosity of hydrophilic organic compound (B) when it is in a molten state.

Known unsaturated fatty acids can be used for unsaturated fatty acid (C-1). Examples include monounsaturated fatty acids such as crotonic acid, myristoleic acid, palmitoleic acid, sapienic acid, elaidic acid, vaccenic acid, paullinic acid, oleic acid, gadoleic acid, eicosenoic acid, erucic acid, and nervonic acid; and polyunsaturated fatty acids such as linoleic acid, conjugated linoleic acid, γ-linolenic acid, eicosadienoic acid, dihomo-γ-linolenic acid, arachidonic acid, α-linolenic acid, pinolenic acid, stearidonic acid, α-eleostearic acid, β-eleostearic acid, mead acid, adrenic acid, eicosatrienoic acid, eicosapentaenoic acid, docosapentaenoic acid, docosahexaenoic acid, tetracosapentaenoic acid, tetracosahexaenoic acid, bosseopentaenoic acid, osbond acid, sardine acid, and herring acid. In view of reducing the hygroscopicity of the dental glass composition, preferred as unsaturated fatty acid (C-1) are unsaturated fatty acids having 8 to 30 carbon atoms, more preferably unsaturated fatty acids having 10 to 25 carbon atoms, even more preferably unsaturated fatty acids having 12 to 20 carbon atoms, particularly preferably polyunsaturated fatty acids having 12 to 20 carbon atoms. In this specification, the term "polyunsaturated" refers to having a degree of unsaturation (carbon-carbon double bond) of 2 or more, without any particular limitation.

Examples of the unsaturated fatty acid ester (C-2) include esters of the aforementioned unsaturated fatty acids, and mixtures of these. In view of reducing the hygroscopicity of the dental glass composition, preferred as unsaturated fatty acid ester (C-2) are unsaturated fatty acid esters having 8 to 30 carbon atoms, more preferably unsaturated fatty acid esters having 10 to 25 carbon atoms, even more preferably unsaturated fatty acid esters having 12 to 20 carbon atoms, particularly preferably polyunsaturated fatty acid esters having 12 to 20 carbon atoms.

The unsaturated fatty acid component (C) helps improve the dispersibility of glass powder (A) and hydrophilic organic compound (B) due to its compatibility or solubility with the hydrophilic organic compound (B) when the hydrophilic organic compound (B) is in a molten state. By the presence of unsaturated fatty acid component (C), it is also possible to adjust the viscosity of the hydrophilic organic compound (B) when it is in a molten state, enhancing the dispersibility of glass powder (A). This enables uniform coloring with good workability, providing even superior glide during drawing. By enhancing the dispersibility of glass powder (A) and hydrophilic organic compound (B) with its presence, the unsaturated fatty acid component (C) can integrate with the other components, including the hydrophilic organic compound (B) in the binder, improving the fixability of the dental glass composition even more greatly while exhibiting excellent chromogenicity. Among the different types of unsaturated fatty acid components (C) above, preferred is unsaturated fatty acid (C-1) for its superior ability to produce these effects.

The unsaturated fatty acid component (C) in the present invention must be sufficiently burned off to such an extent that it does not affect the color after firing when the dental glass composition is coated on the base material and the dental porcelain is fired following build-up on the dental glass composition. In this respect, the boiling point is preferably 400°C or less, more preferably 385°C or less, even more preferably 370°C or less. The boiling point is preferably 100°C or more because the unsaturated fatty acid component (C), when its boiling point is less than 100°C, undergoes evaporation and drying even at room temperature, making it difficult to stably maintain the solid form. The boiling point of unsaturated fatty acid component (C) is preferably 100 to 400°C, more preferably 100 to 385°C, even more preferably 100 to 370°C.

When the binder comprises unsaturated fatty acid component (C), the content of unsaturated fatty acid component (C) is preferably 0.5 to 30 mass%, more preferably 1.0 to 25 mass%, even more preferably 1.5 to 20 mass%, particularly preferably 2.0 to 18 mass% in the total amount of the dental glass composition. When the content of unsaturated fatty acid component (C) is within these ranges, it is easier to impart adequate strength and heat resistance to the dental prostheses produced, and to increase the glide and fixability of the dental glass composition during drawing, in addition to providing excellent chromogenicity.

When the binder comprises a wax component (D) in addition to hydrophilic organic compound (B) and unsaturated fatty acid component (C), the content of unsaturated fatty acid component (C) is preferably 0.2 to 30 mass%, more preferably 0.5 to 25 mass%, even more preferably 1.0 to 20 mass%, particularly preferably 1.5 to 18 mass% in the total amount of the dental glass composition.

When the binder comprises unsaturated fatty acid component (C) in the present invention, the content of unsaturated fatty acid component (C) is preferably the same or lower than the content of hydrophilic organic compound (B), more preferably lower than the content of hydrophilic organic compound (B), regardless of whether the binder comprises the wax component (D) in addition to the hydrophilic organic compound (B).

A certain preferred embodiment is, for example, a dental glass composition in which the binder comprises a hydrophilic organic compound (B) that is solid at 25°C, and an unsaturated fatty acid component (C) that is liquid at 25°C.

### <Wax Component (D)>

In a dental glass composition of the present invention, it is preferable that the binder further comprise a wax component (D) that is solid at 25°C. By incorporating a wax component (D), the wax component (D) becomes integrated with binders other than wax component (D), facilitating the solidification of the dental glass composition at ordinary temperature. With the wax component (D), the dental glass composition can also have reduced hygroscopicity, exhibiting excellent stability in its properties during prolonged storage. The wax component (D) may be used alone, or two or more thereof may be used in combination. The wax component (D) has a solubility of less than 1 mass% in water at 25°C.

In view of achieving excellent stability in properties during prolonged storage at room temperature and providing good fixability in dental prostheses, the wax component (D) used in the present invention has a melting point of preferably 35 to 120°C, more preferably 40 to 110°C, even more preferably 50 to 100°C. When the melting point is 35°C or less, it may not be possible to maintain a solid form, raising a possibility of flowing out from the drawn surface while layering the dental glass composition or building up the surface porcelain layer. A melting point higher than 120°C poses a risk of failed adhesion to dental prostheses and inadequate coloring.

The content of wax component (D) is preferably 1 to 55 mass%, more preferably 3 to 50 mass%, even more preferably 5 to 45 mass%, particularly preferably 7 to 35 mass% in the total amount of a dental glass composition of the present invention. When the content of the wax component falls within these ranges, the wax component becomes integrated with the other binders, enhancing chromogenicity or stainability. This makes it easier to provide a solid dental glass composition that possesses high formability and strength, and good adhesion and fixability to dental prostheses.

In certain embodiments, it is preferable that the binder in a dental glass composition of the present invention comprise a wax component with a melting point of less than 75°C (hereinafter, also referred to as "first wax (D-1)"). By incorporating a first wax (D-1) in the dental glass composition, the dental glass composition exhibits even superior glide during drawing. By incorporating a first wax (D-1), the dental glass composition can exhibit even superior glide during drawing, particularly when the dental glass composition does not comprise the unsaturated fatty acid component (C).

The first wax (D-1) has a melting point of preferably 50°C or more and less than 75°C, more preferably 55 to 73°C, even more preferably 60 to 70°C.

Preferred examples of first wax (D-1) include white petroleum (melting point: approximately 38 to 60°C), stearic acid (melting point: approximately 69 to 70°C), candelilla wax (melting point: approximately 68 to 72°C), beeswax (melting point: approximately 63°C), hardened palm oil (melting point: approximately 59°C), microcrystalline wax (melting point: approximately 67 to 70°C), paraffin wax (melting point: approximately 47 to 69°C), hardened beef tallow (melting point: approximately 46°C), and ketone wax (melting point: approximately less than 60 to 75°C).

In certain embodiments, the content of first wax (D-1) is preferably 1 to 55 mass%, more preferably 1.5 to 50 mass%, even more preferably 5 to 45 mass%, particularly preferably 7 to 35 mass% in the total amount of the dental glass composition. With the content of first wax (D-1) falling in these ranges, it is possible to provide a dental glass composition that exhibits excellent stability in its properties during prolonged storage at room temperature, and even superior glide during drawing.

When a dental glass composition of the present invention comprises the unsaturated fatty acid component (C) and/or first wax (D-1), there is a possibility where the dental glass composition adhering to dental prostheses becomes easily detached due to forces such as rubbing, though the dental glass composition exhibits improved smoothness or glide during drawing. In view of enhancing the fixability of the dental glass composition, it is preferable to comprise a high-melting-point wax (D-2) (hereinafter, also referred to as "second wax (D-2)"), together with the unsaturated fatty acid component (C) and/or first wax (D-1).

In this specification, the second wax (D-2) refers to waxes with a melting point of 75 to 120°C. A second wax (D-2) with a melting point of 75°C or more provides conformability to the fine surface irregularities of the base material when combined with unsaturated fatty acid component (C) and/or first wax (D-1), potentially increasing the fixability of the dental glass composition.

Examples of the second wax (D-2) include carnauba wax, hardened castor oil (melting point: approximately 80 to 90°C), polyolefin wax (with a melting point of 75°C or more, for example, such as low-molecular polyethylene wax (melting point: approximately 100 to 120°C)), long-chain saturated fatty acids (such as behenic acid), saturated fatty acid amides (such as stearamide; a melting point of approximately 98 to 105°C), and microcrystalline wax (melting point: approximately 75 to 98°C). Preferred are behenic acid and carnauba wax for advantages such as high melting point, the ability to solidify in small quantities, and leaving no burn residue after firing.

The second wax (D-2) accounts for preferably 3 to 55 mass%, more preferably 5 to 50 mass%, even more preferably 10 to 45 mass%, particularly preferably 15 to 35 mass% of the wax component (D). With the content of second wax (D-2) falling in these ranges, it is easier to achieve a balance between the smoothness of the dental glass composition during drawing and the fixability of the dental glass composition. In certain embodiments, the content of second wax (D-2) is preferably lower than that of first wax (D-1).

In view of providing even superior smoothness in the dental glass composition during drawing and greater fixability for the dental glass composition, the content of second wax (D-2) in the dental glass composition containing second wax (D-2) is preferably less than 15 mass%, more preferably less than 12 mass%, even more preferably less than 10 mass% in the total amount of the dental glass composition.

The wax component (D) in the present invention must be sufficiently burned off to such an extent that it does not affect the color after firing when the dental glass composition is coated on the base material and the dental porcelain is fired following build-up on the dental glass composition. In this respect, the boiling point is preferably 400°C or less, more preferably 385°C or less, even more preferably 370°C or less.

A certain preferred embodiment is, for example, a dental glass composition in which the binder comprises a hydrophilic organic compound (B) that is solid at 25°C, an unsaturated fatty acid component (C) that is liquid at 25°C, and a wax component (D).

Another preferred embodiment is, for example, a dental glass composition in which the binder comprises a hydrophilic organic compound (B) that is solid at 25°C, and a wax component (D).

Yet another preferred embodiment is, for example, a dental glass composition in which the binder consists of a hydrophilic organic compound (B) that is solid at 25°C.

Still another preferred embodiment is, for example, a dental glass composition in which the binder consists of a hydrophilic organic compound (B) that is solid at 25°C, and an unsaturated fatty acid component (C) that is liquid at 25°C.

Yet another preferred embodiment is, for example, a dental glass composition in which the binder consists of a hydrophilic organic compound (B) that is solid at 25°C, and a wax component (D).

A certain preferred embodiment is, for example, a dental glass composition in which the binder consists of a hydrophilic organic compound (B) that is solid at 25°C, an unsaturated fatty acid component (C) that is liquid at 25°C, and a wax component (D).

In another certain preferred embodiment, the total content of glass powder (A) and the binder is preferably 90 mass% or more in the total amount of the dental glass composition. In view of even superior chromogenicity and greater fixability to dental prostheses, the total content of glass powder (A) and the binder is more preferably 95 mass% or more, even more preferably 97 mass% or more, particularly preferably 98 mass% or more.

A dental glass composition of the present invention may optionally comprise known additives used for dental glass composition applications. Examples of such additives include resins (for example, ethylene-vinyl acetate copolymer), adhering agents, antioxidants, surfactants, preservatives, anti-fungal agents, and antimicrobial agents. The additives may be used alone, or two or more thereof may be used in combination.

A dental glass composition of the present invention may optionally comprise adhesive resins (known adhesive resins, for example, rosin, and/or modified rosins such as petroleum resin). Examples of modified rosins include esters such as rosin esters, hydrogenated rosin esters, and/or polymerized rosin esters.

A dental glass composition of the present invention can be produced using a glass powder (A) and a hydrophilic organic compound (B), using a known method. A dental glass composition of the present invention can also be produced using a known method when it comprises an unsaturated fatty acid component (C) and a wax component (D). The method of production is not particularly limited. For example, a dental glass composition of the present invention can be produced by adding and kneading a glass powder (A) with a hydrophilic organic compound (B), an unsaturated fatty acid component (C), and a wax component (D), followed by extrusion molding of the mixture.

In certain embodiments, when the binder components (particularly the hydrophilic organic compound (B) and wax component (D)) have high melting points, a dental glass composition of the present invention can be obtained by a method that involves adding and mixing the glass powder (A) while melting the binder components under heat, and pouring the mixture into a mold and solidifying it by cooling with airflow before removing the resulting solid from the mold. A dental glass composition with evenly dispersed glass powder (A) can be obtained by adding and mixing glass powder (A) into molten binder components, and cooling the mixture after dispersing the glass powder (A).

The mixing of components (preferably, the binder components) excluding the glass powder (A) may be conducted while these are in a liquid state. Typically, for binder components having melting points higher than room temperature, these are mixed in a liquid state after being heated at a temperature equal to or higher than their melting points. The heating temperature for melting the binder components can vary according to the type of binder component, and may be 50°C or more, 80°C or more, 100°C or more, or 150°C or more, though it is not particularly limited.

The sequence of mixing the components is not particularly limited. Preferably, the glass powder (A) is added and dispersed in the liquid state of hydrophilic organic compound (B). When incorporating the unsaturated fatty acid component (C), the unsaturated fatty acid component (C) may be mixed after dispersing the glass powder (A) in hydrophilic organic compound (B). It is, however, preferable to add the glass powder (A) to a mixture of hydrophilic organic compound (B) and unsaturated fatty acid component (C) prepared in advance. Alternatively, the glass powder (A) may be added and mixed into a partial mixture of hydrophilic organic compound (B) and unsaturated fatty acid component (C), and the remaining portions of hydrophilic organic compound (B) and unsaturated fatty acid component (C) may be added after further mixing. A solid dental glass composition can be obtained by cooling the constituent components in the mixture inside a mold, at a temperature below the melting point of the hydrophilic organic compound (B).

A dental glass composition of the present invention may additionally comprise other components (optional components), provided that such additional components do not hinder the effectiveness of the present invention, and that the dental glass composition comprises the glass powder (A), and the hydrophilic organic compound (B) that is solid at 25°C, and in which a solvent is essentially absent.

Examples of such components include colorants, pH adjusters, polymerization accelerators, and polymerization initiators. Examples of the colorants include colorants that decolor during firing.

Examples of the colorants that decolor during firing include food dyes that dissolve in organic solvent. Examples of such food dyes include:
organic dyes with two or more aromatic groups, such as yellow 4 (tartrazine), yellow 5 (sunset yellow FCF), red 2 (amaranth), red 102 (new coccin), blue 1 (brilliant blue FCF), blue 2 (indigo carmine), and green 3 (fast green FCF);
organic dyes (xanthene dyes) containing fused aromatic groups with a xanthene core, such as acid red 289, bromopyrogallol red, rhodamine B, rhodamine 6G, rhodamine 6GP, rhodamine 3GO, rhodamine123, eosin, eosin B, eosin Y, fluorescein, and fluorescein isothiocyanate;
cochineal dyes (carmic acid dyes); and
betalain dyes, such as beet red (main components: isobetanin and betanin), betanin, isobetanin, probetanin, and neobetanin.

A certain embodiment is, for example, a dental glass composition that comprises a glass powder (A), a hydrophilic organic compound (B), and, optionally, an unsaturated fatty acid component (C), and that is essentially free of a colorant that decolors during firing. Referring to the phrase "essentially free of a colorant that decolors during firing", the content of the colorant that decolors during firing may be, for example, 1,000 ppm or less by mass, preferably 100 ppm or less by mass, more preferably 10 ppm or less by mass, even more preferably 1 ppm or less by mass, particularly preferably 0.1 ppm or less by mass, most preferably 0 ppm by mass with respect to the mass of the dental glass composition. The content of the additional components is not particularly limited, and is preferably 15.0 mass% or less, more preferably 12.0 mass% or less.

Concerning a dental prosthesis producing method of the present invention, an example method of producing a dental prosthesis involves the steps of applying the dental glass composition to a dental prosthesis base material having a ceramic surface, and conducting firing after building up dental porcelain, without firing the base material following the application of the dental glass composition and before building up dental porcelain. The base material is as described above. The dental prosthesis base material having a ceramic surface refers to a base material (such as a core or a frame) before build-up of dental porcelain.

Concerning a dental prosthesis producing method of the present invention, another example method of producing a dental prosthesis involves the steps of applying the dental glass composition to a dental prosthesis base material having a ceramic surface, and conducting firing after building up dental porcelain, without firing the base material following the application of the dental glass composition. The base material is as described above. The dental prosthesis base material having a ceramic surface refers to a base material (such as a core or a frame) before build-up of dental porcelain.

The dental prosthesis base material is not particularly limited, as long as it is a material having dental use. Examples include various types of ceramics (dental ceramics such as zirconium oxide (zirconia), aluminum oxide (alumina), feldspar glass, and lithium disilicate), and other types of ceramics, including metal-ceramic dental prostheses and all-ceramic dental prostheses prepared by fusing dental porcelain onto dental metal cores or cores made of the aforementioned ceramics, either in single or multiple layers to express the dentin or enamel shade.

The dental prosthesis base material may be entirely ceramic, as long as it at least partially contains ceramic, and has a ceramic surface.

A dental prosthesis base material with a ceramic surface can be produced by processing, for example, a commercially available product (for example, KATANA^{®} zirconia STML, manufactured by Kuraray Noritake Dental Inc.) into a dental prosthesis of a desired shape using a known method.

The method of applying the dental glass composition to a dental prosthesis base material having a ceramic surface is not particularly limited, and this may be achieved using known methods, devices, or instruments.

A dental glass composition of the present invention can used as a porcelain stain for internal staining or surface staining to achieve staining of a base material upon firing after being directly applied to a dental prosthesis base material having a ceramic surface. When the dental prosthesis base material with a ceramic surface is highly transparent zirconia that has come to be used in recent years, a glass composition of the present invention may be used as, for example, a porcelain stain for internal staining to achieve staining upon firing after being directly applied to zirconia by assuming that the zirconia frame itself has the dentin shade.

In this specification, the term "direct application" refers to the act of application in the original form, without undergoing paste preparation with a designated liquid (a liquid material containing, for example, water or ethanol).

Another aspect of the present invention provides use where a dental glass composition that comprises a glass powder (A), a hydrophilic organic compound (B) that is solid at 25°C, an unsaturated fatty acid component (C), and a wax component (D), and that is essentially free of water and propylene glycol serves as a direct-application porcelain stain.

A dental glass composition of the present invention may have a solid form at 25°C. A dental glass composition of the present invention can be used as a porcelain stain for direct application, even when it is solid at ordinary temperature.

In this specification, "ordinary temperature" refers to the temperature range of 20°C ± 15°C specified by JIS Z 8703-1983, and may be, for example, 25°C.

A dental glass composition of the present invention allows for easy drawing of tooth features (hereinafter, also referred to as "characters"), mimicking their appearance, on a dental prosthesis base material having a ceramic surface. Specifically, a dental glass composition of the present invention can be used on its own in ways similar to drawing with wood charcoal or oil pastels, or can be sharpened to a pencil-like point to enable easy drawing of fine characters such as enamel cracks or hairlines. A dental glass composition of the present invention can also extend its use to various techniques, such as applying it to a cheek brush or a spongy eye color tip applicator, and rubbing it onto a dental prosthesis base material having a ceramic surface to draw color with gradients (gradation or blurring). The term "character" refers to, for example, the white band of natural teeth, the coloration or discoloration of cervical region and adjacent surfaces, as well as demineralization, enamel cracks, and hairlines infiltrated by coloring components.

Traditional porcelain stains require fixation firing after application. Specifically, when using traditional porcelain stains, fixation firing must be conducted after the porcelain stain is applied, before building up the porcelain on the surface layer, in order to prevent the porcelain stain from bleeding or flowing out. Moreover, in order to mimic the dentin or other internal structures of natural teeth, it is necessary to vertically apply stains from the incisal edge toward the cervical region of dental prostheses, whereas the porcelain stain must be mesiodistally layered sideways to express features such as the white band. Each change in the staining direction necessitates fixation firing to prevent the colors from blending together.

In contrast, a dental glass composition of the present invention, unlike traditional porcelain stains, is solid and is not flowable, demonstrating excellent fixability after drawing, and eliminating the need for fixation firing after each layering.

Furthermore, a dental glass composition of the present invention exhibits superior fixability to the final dental prosthesis even when dental porcelain is built up on the surface layer without firing and fixing the dental glass composition after application, preventing bleeding or flowing. Additionally, there is no detachment, bubbling, or blackening at the interface between the porcelain and the base material during subsequent firing after the build-up of dental porcelain, simplifying the fixation firing process. It is therefore possible with a dental prosthesis producing method of the present invention to produce a dental prosthesis with a single firing process performed after dental porcelain is built up following the application of the dental glass composition to the base material. This simplifies the production process and greatly improves production efficiency.

In the case of the internal staining method, the dental prosthesis is finished through the process where dental porcelain is built up and fired after applying a dental glass composition of the present invention, and the outer shape is adjusted to create a crown shape matching natural teeth before melting only the dental porcelain surface layer to provide glaze (self-glazing)

A dental glass composition of the present invention is also applicable to the surface staining method, where staining is performed concurrently with glazing employing low-melting-temperature glaze glass to provide glaze, after the final adjustment of the external shape.

The dental porcelain that can be used for a dental prosthesis producing method of the present invention is not particularly limited, and known dental porcelain may be used.

Examples of known dental porcelain include commercially available products, for example, such as Cerabien^{®} ZR internal stain / external stain, Cerabien^{®} ZR press LF internal stain / external stain (manufactured by Kuraray Noritake Dental Inc.).

In the present invention, the firing temperature (highest firing temperature) in the firing step after the build-up of dental porcelain can appropriately vary according to factors such as the type of dental porcelain, and a form of use. Accordingly, the firing temperature is not particularly limited, as long as it allows the colorant (for example, inorganic pigment) to exhibit color. However, the firing temperature is preferably 700°C or more, more preferably 730°C or more, even more preferably 750°C or more. The upper limit of firing temperature is preferably 1,100°C or less, more preferably 1,050°C or less, even more preferably 1,000°C or less, though it is not particularly limited. The rate of temperature increase to the highest firing temperature during firing can appropriately vary according to the type of porcelain. However, the preferred rate of temperature increase is about 10 to 70°C/min, more preferably about 20 to 60°C/min, though it is not particularly limited.

Examples of the final dental prostheses that can be obtained using a glass composition of the present invention include metal-ceramic dental prostheses, and all-ceramic dental prostheses restored with materials such as alumina cores or zirconia cores.

Specific examples of the final dental prostheses include copings, frameworks, denture bases, dentures, orthodontic products, and implant products.

Examples of the dentures include crowns, bridges, inlays, onlays, and laminate veneers.

Examples of the orthodontic products include brackets.

Examples of the dental implant products include implants, abutments, implant fixtures, implant bridges, implant burs, and implant superstructures that include gum (gingival) sections attached to the prosthesis.

### EXAMPLES

The following describes the present invention in greater detail through Examples. The present invention, however, is not limited to the Examples below.

The solid dental glass compositions of Examples 1 to 14 and the glass compositions of Comparative Examples 1 to 9 were prepared as follows, and their properties were evaluated. The results are presented in Tables 1 and 2.

In Tables 1 and 2, "%" represents the mass percentage of each component.

In Tables 1 and 2, "PEG-2000" and "PEG-1000" refer to polyethylene glycol with a weight-average molecular weight (Mw) of 2,000, and polyethylene glycol with a weight-average molecular weight (Mw) of 1,000, respectively.

In Tables 1 and 2, "Macrogol paste" refers to a mixture of polyethylene glycol 400 (Mw: 400) and 4000 (Mw: 4000) in equal amounts.

In Tables 1 and 2, "ordinary temperature" indicates evaluations conducted at 25°C. The properties of the compositions were also evaluated at 25°C.

The weight-average molecular weight was determined through calculation by gel permeation chromatography (GPC) in terms of polystyrene.

PEG-2000 had a solubility of 50 mass% or more in water at 25°C. PEG-1000 had a solubility of 50 mass% or more in water at 25°C. The macrogol paste had a solubility of 50 mass% or more in water at 25°C. The stearic acid, white petroleum, carnauba wax, and behenic acid all had a solubility of less than 1 mass% in water at 25°C.

### [Examples 1 to 14 and Comparative Examples 1 to 6, and 9]

To achieve the mass percentages listed in Tables 1 to 2, the hydrophilic organic compound (B), unsaturated fatty acid component (C), and wax component (D) were melted in a container on a hot stirrer at 120°C. Subsequently, the glass powder (A) (Cerabien^{®} ZR internal stain, manufactured by Kuraray Noritake Dental Inc.) was added and mixed until a uniform mixture was obtained, using a metal spatula. The mixture was poured into a mold measuring 20.0 mm in length, 3.0 mm in width, and 3.0 mm in depth, and allowed to cool and solidify to yield a solid dental glass composition. The glass powder (A) used is a potassium aluminosilicate glass product, containing pigments and appearing reddish brown in color.

The average particle diameter of glass powder (A) was determined as the average particle diameter based on the median diameter (d₅₀), where cumulative volume frequency in the volumetric particle size distribution reaches 50% from the smaller diameter side, using a laser diffraction particle size distribution analyzer (SALD-2300, manufactured by Shimadzu Corporation).

### [Comparative Examples 7 and 8]

The components listed in Table 2 were weighed on a glass plate to achieve the mass percentages indicated in Table 2, and directly mixed with a spatula to obtain paste-like compositions.

### [Preparation of Zirconia Sintered Body]

A commercially available dental zirconia (KATANA^{®} zirconia STML, manufactured by Kuraray Noritake Dental Inc.) was processed into a cylindrical shape with a diameter of approximately 18 mm, and subjected to firing with a two-hour retention period at a maximum temperature of 1,550°C using a Noritake KATANA^{®} F-1 furnace manufactured by SK Medical Electronics Co., Ltd., producing a zirconia sintered body that served as a base material.

### [Method of Fixability Evaluation]

The dental glass composition of each Example and Comparative Example prepared in the manner described above was used to draw a line on the surface of the base-material zirconia sintered body prepared using the above method. Subsequently, a kneaded mixture of dental porcelain (Cerabien^{®} ZR translucent Tx, manufactured by Kuraray Noritake Dental Inc.) in purified water was built up thereon.

### <Evaluation Criteria>

The dental glass composition was rated "a" when the shape of the drawn line remained intact in all of its samples, whereas the dental glass composition was rated "b" when the shape of the drawn line was preserved but there was blending of colors in any of the samples, and "c" when any of the samples showed the drawn line flowing out with the porcelain, and was unable to maintain the shape of the line (n = 3). In practical terms, the ratings "a" and "b" were deemed acceptable.

### [Method of Chromogenicity Evaluation]

The dental glass composition of each Example and Comparative Example prepared in the manner described above was used to draw a line on the surface of the base-material zirconia sintered body prepared using the above method. Subsequently, a kneaded mixture of dental porcelain (Cerabien^{®} ZR translucent Tx, manufactured by Kuraray Noritake Dental Inc.) in purified water was built up thereon, followed by firing at a maintained temperature of 930°C for 1 minute.

### <Evaluation Criteria>

The dental glass composition was rated "a" when the drawn line showed a clear color in all of its samples subjected to visual examination, whereas the dental glass composition was rated "b" when the color was unclear and had a white hue in any of the samples, and "c" when any of the samples showed bleeding of color or no color at all (n = 3). In practical terms, the ratings "a" and "b" were deemed acceptable.

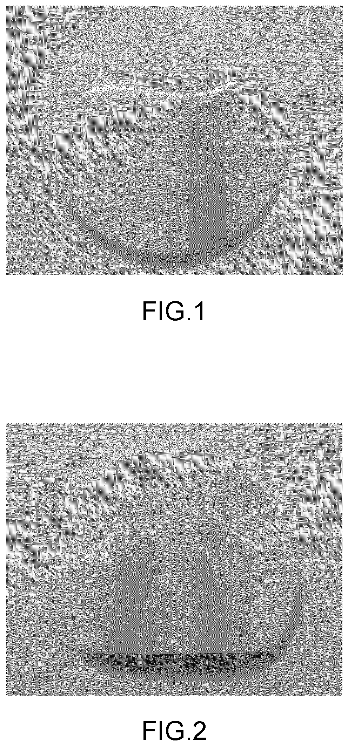

FIG. 1 depicts a photograph showing a specimen subjected to fixability and chromogenicity evaluation according to Example 1. FIG. 2 depicts a photograph showing a specimen subjected to fixability and chromogenicity evaluation according to Comparative Example 5. As depicted in FIG. 2, the drawn line failed to maintain its shape, and showed color bleeding in Comparative Example 5. In contrast, the drawn line in FIG. 1 maintained its shape, and showed a clear color.

### [Method of Evaluation of Interface Condition]

Samples assessed according to the method described in the foregoing section [Method of Chromogenicity Evaluation] were cut perpendicular to the drawn line, and the cut surface was polished to provide a smooth surface.

The polished cut surface was observed with a light microscope (DIGITAL MICROSCOPE KH-7700, Hirox Co., Ltd.) at a magnification of 1,000 times to check for notable bubbles or detachment in the dental glass composition at the interface between the base material and dental porcelain.

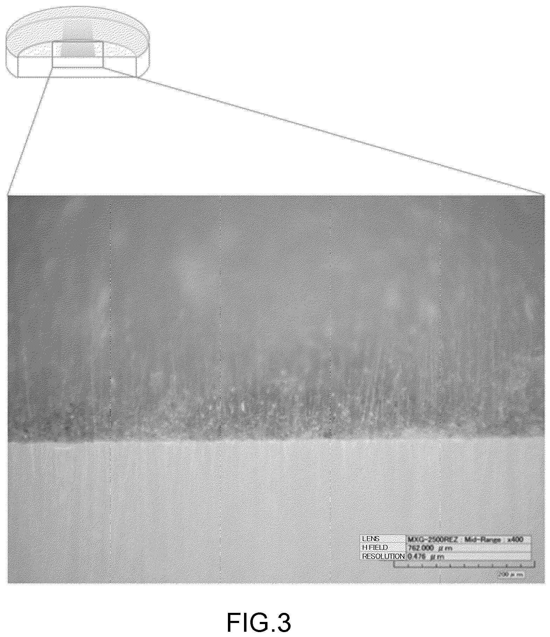

As a control, dental porcelain was built up and fired on the base material following the conventional internal staining technique (with fixation firing). Subsequent observation of the cut surface revealed the presence of bubbles, though minor. FIG. 3 shows the cut surface relevant to the evaluation method, along with the result of observing the control cut surface with a light microscope (picture). Because the sole presence of fine bubbles, such as those shown in FIG. 3, does not necessarily lead to detachment, the interface was evaluated based on the following criteria (n = 3).

### <Evaluation Criteria>

Samples were rated "a" when the level of bubbles was comparable to that of the control in all samples, "b" when there were more bubbles than in the control in any of the samples, "c" when there were notably more bubbles than in the control, or any instance of interface delamination was observed in any of the samples, and "d" when interface delamination was observed in several locations in any of the samples. In practical terms, the ratings "a" and "b" were deemed acceptable.

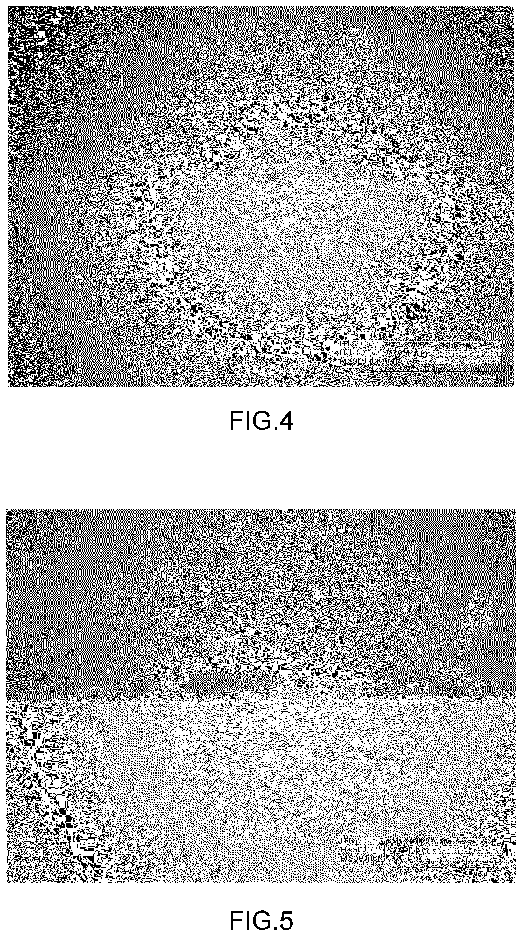

FIG. 4 shows a light micrograph of the cut surface in the evaluation of interface condition according to Example 1. FIG. 5 shows a light micrograph of the cut surface in the evaluation of interface condition according to Comparative Example 2. In FIG. 5, an air layer is present between the base material and porcelain, confirming distinct detachment.

### [Method of Workability Evaluation]

The dental glass compositions prepared using the method described above were used to draw lines on the surface of zirconia sintered bodies prepared according to the method described in the foregoing section [Preparation of Zirconia Sintered Body].

### <Evaluation Criteria>

The dental glass compositions were rated "a" when lines were drawable and uniformly spreadable in all of the samples, "b" when lines were drawable but were not uniform throughout in any of the samples, and "c" when any part of the zirconia sintered body surface was exposed in any of the samples (n = 3). In practical terms, the ratings "a" and "b" were deemed acceptable.

**[Table 1]**

| | | | | | | | Examples | | | | | | |
|---|---|---|---|---|---|---|---|---|---|---|---|---|---|
| | | | | | | | 1 | 2 | 3 | 4 | 5 | 6 | 7 |
| | | Average particle diameter (d50) | properties (ordinary temperature) | Solubility (water) | Melting point (°C) | Boiling point (°C) | | | | | | | |
| A Glass powder | CZR IS Reddish Brown | 4.0 µm | | | | | 85% | 70% | 60% | 56% | 70% | 40% | 50% |
| | | 350 µm | | | | | | | | | | | |
| B Hydrophilic organic compound | PEG-2000 | | Solid | Soluble | 58 | 206 | | | 10% | 26% | | 18% | 15% |
| B Hydrophilic organic compound | PEG-1000 | | Solid | Soluble | 40 | >200 | | | 20% | | | | |
| B Hydrophilic organic compound | Macrogol paste | | Paste | Soluble | 57 | >200 | 12% | 20% | | | 15% | | |
| C Unsaturated fatty acid component | Linoleic acid | | Liquid | Insoluble | -5 | 229 | 3% | 10% | 10% | 18% | 6% | 12% | 10% |
| D-1 First wax component | Stearic acid | | Solid | Essentially insoluble | 69-70 | 361 | | | | | 9% | | |
| D-1 First wax component | White petroleum | | Paste | Insoluble | 38-60 | 302 | | | | | | 30% | 25% |
| D-2 Second wax component | Carnauba wax | | Solid | Insoluble | 81-86 | | | | | | | | |
| D-2 Second wax component | Behenic acid | | Solid | Insoluble | 80 | 306 | | | | | | | |
| Additional liquid component | Oleyl alcohol | | Liquid | Insoluble | 13-19 | 330 | | | | | | | |
| Additional liquid component | Liquid paraffin | | Liquid | Insoluble | -17.8 | 360 | | | | | | | |
| Organic solvent | Propylene glycol | | Liquid | Soluble | -59 | 189 | | | | | | | |
| Water | Purified water | | Liquid | Soluble | 0 | 100 | | | | | | | |
| | | | | | Total | | 100% | 100% | 100% | 100% | 100% | 100% | 100% |
| Composition properties | | | | | | | Solid | Solid | Solid | Solid | Solid | Solid | Solid |
| Workability | | | | | | | b | a | a | b | a | b | b |
| Fixability | | | | | | | b | a | b | b | a | b | b |
| Chromogenicity | | | | | | | a | a | a | b | a | b | b |
| Interface condition | | | | | | | b | a | a | a | a | b | a |

**[Table 1] Continued**

| | | | | | | | Examples | | | | | | |
|---|---|---|---|---|---|---|---|---|---|---|---|---|---|
| | | | | | | | 8 | 9 | 10 | 11 | 12 | 13 | 14 |
| | | Average particle diameter (d50) | properties (ordinary temperature) | Solubility (water) | Melting point (°C) | Boiling point (°C) | | | | | | | |
| A Glass powder | CZR IS Reddish Brown | 4.0 µm | | | | | 60% | 70% | 80% | | 60% | 70% | 60% |
| | | 350 µm | | | | | | | | 70% | | | |
| B Hydrophilic organic compound | PEG-2000 | | Solid | Soluble | 58 | 206 | 12% | 9% | 6% | 9% | 12% | 9% | |
| B Hydrophilic organic compound | PEG-1000 | | Solid | Soluble | 40 | >200 | | | | | | | |
| B Hydrophilic organic compound | Macrogol paste | | Paste | Soluble | 57 | >200 | | | | | | | 40% |
| C Unsaturated fatty acid component | Linoleic acid | | Liquid | Insoluble | -5 | 229 | 8% | 6% | 4% | 6% | 12% | 9% | |
| D-1 First wax component | Stearic acid | | Solid | Essentially insoluble | 69-70 | 361 | | | | | | | |
| D-1 First wax component | White petroleum | | Paste | Insoluble | 38-60 | 302 | 20% | 15% | 10% | 15% | 12% | 9% | |
| D-2 Second wax component | Carnauba wax | | Solid | Insoluble | 81-86 | | | | | | 4% | 3% | |
| D-2 Second wax component | Behenic acid | | Solid | Insoluble | 80 | 306 | | | | | | | |
| Additional liquid component | Oleyl alcohol | | Liquid | Insoluble | 13-19 | 330 | | | | | | | |
| Additional liquid component | Liquid paraffin | | Liquid | Insoluble | -17.8 | 360 | | | | | | | |
| Organic solvent | Propylene glycol | | Liquid | Soluble | -59 | 189 | | | | | | | |
| Water | Purified water | | Liquid | Soluble | 0 | 100 | | | | | | | |
| | | | | | Total | | 100% | 100% | 100% | 100% | 100% | 100% | 100% |
| Composition properties | | | | | | | Solid | Solid | Solid | Solid | Solid | Solid | Solid |
| Workability | | | | | | | a | a | b | a | a | a | b |
| Fixability | | | | | | | a | a | b | a | a | a | b |
| Chromogenicity | | | | | | | a | a | b | a | a | a | b |
| Interface condition | | | | | | | a | a | a | a | a | a | a |

**[Table 2]**

| | | | | | | | Comparative Examples | | | | | | | | |
|---|---|---|---|---|---|---|---|---|---|---|---|---|---|---|---|
| | | | | | | | 1 | 2 | 3 | 4 | 5 | 6 | 7 | 8 | 9 |
| | | Average particle diameter (d50) | properties (ordinary temperature) | Solubility (water) | Melting point (°C) | Boiling point (°C) | | | | | | | | | |
| A Glass powder | CZR IS Reddish Brown | 4.0 µm | | | | | 70% | 68% | 60% | 50% | 60% | 80% | 65% | 65% | 65% |
| | | 350 µm | | | | | | | | | | | | | |
| B Hydrophilic organic compound | PEG-2000 | | Solid | Soluble | 58 | 206 | | | | | 20% | 10% | | | 10% |
| B Hydrophilic organic compound | PEG-1000 | | Solid | Soluble | 40 | >200 | | | | | | | | | |
| B Hydrophilic organic compound | Macrogol paste | | Paste | Soluble | 57 | >200 | | | | | | | | | |
| C Unsaturated fatty acid component | Linoleic acid | | Liquid | Insoluble | -5 | 229 | 6% | | 12% | | | | | | |
| D-1 First wax component | Stearic acid | | Solid | Essentially insoluble | 69-70 | 361 | 9% | 4% | 12% | 30% | | | | | |
| D-1 First wax component | White petroleum | | Paste | Insoluble | 38-60 | 302 | 15% | 14% | 12% | | | | | | |
| D-2 Second wax component | Carnauba wax | | Solid | Insoluble | 81-86 | | | 2% | 4% | | | | | | |
| D-2 Second wax component | Behenic acid | | Solid | Insoluble | 80 | 306 | | 8% | | | | | | | |
| Additional liquid component | Oleyl alcohol | | Liquid | Insoluble | 13-19 | 330 | | | | 20% | | | | | |
| Additional liquid component | Liquid paraffin | | Liquid | Insoluble | -17.8 | 360 | | 4% | | | | | | | |
| Organic solvent | Propylene glycol | | Liquid | Soluble | -59 | 189 | | | | | 20% | 10% | 35% | | |
| Water | Purified water | | Liquid | Soluble | 0 | 100 | | | | | | | | 35% | 25% |
| | | | | | Total | | 100% | 100% | 100% | 100% | 100% | 100% | 100% | 100% | 100% |
| Composition properties | | | | | | | Solid | Solid | Solid | Solid | Paste | Paste | Paste | Paste | Paste |
| Workability | | | | | | | a | a | a | b | b | b | a | a | a |
| Fixability | | | | | | | a | a | a | b | c | c | c | c | c |
| Chromogenicity | | | | | | | a | b | b | a | c | c | c | c | c |
| Interface condition | | | | | | | c | d | c | d | - | - | - | - | - |

In Comparative Examples 1 to 4 lacking hydrophilic organic compound (B), observation of the cut surface showed instances of detachment, voids, or bubbles in parts of the interface between the substrate (base material) and porcelain. The stains, solidified only with the wax components, showed a state of water repellency (no wettability) on the layered porcelain. This appears to have led to the occurrence of detachment or void or bubble formation due to an air layer remaining at the interface between the substrate and porcelain following the firing of porcelain.

Comparative Examples 5 to 9 representing paste form failed to achieve fixation or produce color without fixation firing, compromising the aesthetic appeal as dental prostheses.

Because paste-like compositions inherently require fixation firing, the compromised aesthetic quality is probably due to a result of the applied paste-like composition shifting away from its intended location by partially mixing with the porcelain built up after the application of the paste-like composition.

In contrast, Examples 1 to 14 representing dental glass compositions comprising hydrophilic organic compound (B) demonstrated good fixability and chromogenicity even without fixation firing. This result confirmed the superior effectiveness of the present invention, particularly from the comparison between Example 5 and Comparative Example 1, and between Example 12 and Comparative Example 3.

The dental glass compositions of Examples 1 to 14 were also shown to undergo no changes over time, demonstrating excellent workability and allowing for uniform application.

### INDUSTRIAL APPLICABILITY

A glass composition of the present invention, in its solid form ensuring good fixability to dental prostheses, undergoes hardly any change over time itself, and can be applied to dental prostheses in thin layers, without bleeding due to its lack of flowability, enabling easy fabrication of dental prostheses in the desired shade with good workability. This makes a glass composition of the present invention suitable for use as a dental glass composition.

A dental glass composition of the present invention, requiring no fixation firing, is useful given an expected increase in the use of dental glass compositions, particularly in response to the continuously growing demand for ceramic dental crowns and the associated increase in individual demand for better aesthetics.

## Claims

1. A dental glass composition comprising a glass powder (A) and a binder,
wherein the binder comprises a hydrophilic organic compound (B) that is solid at 25°C, and
the dental glass composition is essentially free of water and propylene glycol.

2. The dental glass composition according to claim 1, wherein the glass powder (A) has an average particle diameter of 0.05 µm to 50 µm.

3. The dental glass composition according to claim 1 or 2, wherein the hydrophilic organic compound (B) has a melting point of 35°C or more.

4. The dental glass composition according to any one of claims 1 to 3, wherein the hydrophilic organic compound (B) is a water-soluble polymer.

5. The dental glass composition according to any one of claims 1 to 4, wherein the content of the hydrophilic organic compound (B) is 1 to 50 mass%.

6. The dental glass composition according to any one of claims 1 to 5, wherein the binder further comprises an unsaturated fatty acid component (C) that is liquid at 25°C.

7. The dental glass composition according to claim 6, wherein the content of the unsaturated fatty acid component (C) is 0.5 to 30 mass%.

8. The dental glass composition according to any one of claims 1 to 7, wherein the binder further comprises a wax component (D) that is solid at 25°C.

9. The dental glass composition according to any one of claims 1 to 8, wherein the content of the water and propylene glycol is less than 1,000 ppm by mass.

10. The dental glass composition according to any one of claims 1 to 9, which is in solid form at 25°C.

11. The dental glass composition according to any one of claims 1 to 10, which is a porcelain stain.

12. A method for producing a dental prosthesis, comprising the steps of:
applying a dental glass composition of any one of claims 1 to 11 to a dental prosthesis base material having a ceramic surface; and
conducting firing after building up dental porcelain, without firing the base material following the application of the dental glass composition and before building up dental porcelain.

13. The method for producing a dental prosthesis according to claim 12, wherein the firing step after building up dental porcelain involves a firing temperature of 700 to 1,100°C.
